# EUROPEAN PATENT SPECIFICATION

(11) **EP 4 001 434 B1**
(45) Date of publication and mention of the grant of the patent: **02.07.2025**
(21) Application number: 21205380.5
(22) Date of filing: 28.10.2021
(51) Int. Cl.: C12Q 1/6883

(54) **SALIVARY BIOMARKER FOR DIAGNOSING XEROSTOMIA AND USE THEREOF**
SPEICHELBIOMARKER ZUR DIAGNOSE VON XEROSTOMIE UND SEINE VERWENDUNG
BIOMARQUEUR SALIVAIRE POUR DIAGNOSTIQUER LA XÉROSTOMIE ET SON UTILISATION

(30) Priority: 18.11.2020 KR 20200154577; 18.11.2020 KR 20200154578
(43) Date of publication of application: 25.05.2022
(73) Proprietor: Korea Institute of Science and Technology, Seoul 02792 (KR); IUCF-HYU (INDUSTRY-UNIVERSITY COOPERATION FOUNDATION HANYANG UNIVERSITY), Seoul 04763 (KR)
(72) Inventor: HWANG, Kyung-Gyun, Seoul (KR); LEE, Jung Hwa, Seoul (KR); LEE, Ji Eun, Seoul (KR); LEE, Su Jin, Seoul (KR); PARK, Yae Eun, Seoul (KR)
(74) Representative: Hutter, Anton

(56) References cited:
- YOO MIN H. ET AL: "TRPV1 regulates inflammatory process in the tongue of surgically induced xerostomia mouse", HEAD AND NECK., vol. 42, no. 2, 13 November 2019 (2019-11-13), US, pages 198 - 209, XP055907191, ISSN: 1043-3074, Retrieved from the Internet <URL:https://onlinelibrary.wiley.com/doi/full-xml/10.1002/hed.25980> DOI: 10.1002/hed.25980
- KANG S.E ET AL: "Soluble Semaphorin 4D/CD100 Is Increased in the Saliva of Sjögren's Syndrome", ARTHRITIS AND RHEUMATOLOGY, 1 September 2008 (2008-09-01), pages 1 - 3584, XP055907255, Retrieved from the Internet <URL:https://onlinelibrary.wiley.com/doi/epdf/10.1002/art.40700> [retrieved on 20220330]
- ICHIYAMA TOMOKO ET AL: "Expression of aquaporin 3 and 5 as a potential marker for distinguishing dry mouth from Sjögren's syndrome", JOURNAL OF ORAL SCIENCE, vol. 60, no. 2, 1 January 2018 (2018-01-01), JP, pages 212 - 220, XP055907267, ISSN: 1343-4934, DOI: 10.2334/josnusd.17-0150
- NGUYEN CUONG Q ET AL: "Differential gene expression in the salivary gland during development and onset of xerostomia in SjÃ gren's syndrome-like disease of the C57BL/6.NOD-Aec1Aec2 mouse", ARTHRITIS RESEARCH AND THERAPY, BIOMED CENTRAL, LONDON, GB, vol. 11, no. 2, 20 April 2009 (2009-04-20), pages R56, XP021053421, ISSN: 1478-6354, DOI: 10.1186/AR2676
- PARK YUN-JONG ET AL: "Uncovering stem cell differentiation factors for salivary gland regeneration by quantitative analysis of differential proteomes", PLOS ONE, vol. 12, no. 2, 3 February 2017 (2017-02-03), pages e0169677, XP055907332, DOI: 10.1371/journal.pone.0169677
- "Affymetrix GeneChip Human Genome U133 Array Set HG-U133A", GEO,, 11 March 2002 (2002-03-11), XP002254749

## Description

### CROSS-REFERENCE TO RELATED APPLICATIONS

This application is based on and claims priority under 35 U.S.C. §119 to Korean Patent Application Nos. 10-2020-0154577 and 10-2020-0154578, filed on November 18, 2020, in the Korean Intellectual Property Office.

### BACKGROUND

### 1. Field

The present disclosure relates to a salivary biomarker for diagnosing xerostomia, a composition and a kit, each for diagnosing xerostomia using the biomarker, and a method of detecting the biomarker using the composition or kit.

### 2. Description of the Related Art

Function of the salivary gland is affected by several conditions. Xerostomia is a subjective sensation of oral dryness due to a lack of saliva in the mouth. When function of the salivary gland is reduced and lubricating or antibacterial actions are decreased due to xerostomia, dental caries and periodontal disease may appear as secondary diseases in the oral cavity. It is difficult to recognize xerostomia in the early stages of salivary gland hypofunction, and it is difficult to distinguish between transient xerostomia and congenital xerostomia. Therefore, it is difficult to rapidly diagnose xerostomia caused by congenital salivary gland hypofunction. Elderly's xerostomia due to aging is also caused by congenital salivary gland hypofunction. Xerostomia due to salivary gland hypofunction is difficult to completely treat after the disease has progressed, and a treatment method focuses on alleviating the symptoms rather than completely treating the disease.

Xerostomia may occur due to a variety of causes, such as Sjogren's syndrome, anemia, diabetes, aging, use of drugs, neurological diseases, etc. Recently, a method of diagnosing and treating Sjogren's syndrome by detecting a biomarker in a saliva sample of an individual with xerostomia has been developed (US Patent Publication No. 9,833,519B2). However, biomarkers for diagnosing xerostomia have not been developed.

Accordingly, there is a need to develop a biomarker that may diagnose xerostomia rapidly, objectively, simply and inexpensively, and has high diagnostic accuracy and specificity.

### SUMMARY

Provided is use of a composition for diagnosing xerostomia.

Provided is use of a kit for diagnosing xerostomia.

Provided is a method of diagnosing xerostomia.

Additional aspects will be set forth in part in the description which follows and, in part, will be apparent from the description, or may be learned by practice of the presented embodiments of the disclosure.

An aspect provides use of a composition for diagnosing xerostomia, the composition including an agent for measuring an expression level of at least one gene, protein or fragment thereof wherein the at least one gene, protein of fragment thereof is nucleobindin-2 (NUCB2),. The composition further comprises an agent for measuring an expression level of at least one gene, protein or fragment thereof selected from the group consisting of immunoglobulin kappa variable 1-8 (KV108), Golgi apparatus protein 1 (GSLG1), protein FAM3B (FAM3B), mucin-21 (MUC21), Ras-related protein Rab-11A (RB11A), lysozyme C (LYSC), calmodulin-like protein 3 (CALL3), alpha-1B- glycoprotein (A1BG), alpha-1-acid glycoprotein 1 (A1AG1), calreticulin (CALR), plasminogen (PLMN), extracellular matrix protein 1 (ECM1), annexin A6 (ANXA6), WD repeat-containing protein 1 (WDR1), leukocyte elastase inhibitor (ILEU), alpha-2-HS- glycoprotein (FETUA), myeloid cell nuclear differentiation antigen (MNDA), matrix metalloproteinase-9 (MMP9), neutrophil gelatinase-associated lipocalin (NGAL), D-dopachrome decarboxylase (DOPD), neutrophil collagenase (MMP8), programmed cell death 6-interacting protein (PDCD6IP), prostaglandin reductase 1 (PTGR1), FAM3D, Zymogen granule protein 16 homolog B (ZG16B), alpha-1-antitrypsin (A1AT), antithrombin-III (ANT3), ATP synthase subunit alpha (ATPA, mitochondrial), histidine- rich glycoprotein (HRG), histone H1.5 (H15), histone H2B type 1-L (H2B1L), immunoglobulin gamma-1 heavy chain (IGG1), poly(rC)-binding protein 1 (PCBP1), and receptor-type tyrosine-protein phosphatase eta (PTPRJ).

The term "xerostomia (or dry mouth)" refers to a symptom of dry mouth due to reduced saliva flow or various other causes. Xerostomia may be also referred to as salivary gland hypofunction. Xerostomia may be caused by causes such as Sjogren's syndrome, anemia, diabetes, nutrient deficiency, aging, use of drugs (e.g., antihistamines, psychotropic drugs, antihypertensive drugs), nervous system diseases, mental disorders (e.g., depression), radiation therapy, etc. When xerostomia is induced, it may cause difficulty in swallowing food due to the dry sensation itself, and discomfort while speaking, occurrence of tooth decay (dental caries) and periodontitis (periodontal disease) increases and is easy to worsen, fungal infection in the oral cavity, tongue pain, bad breath, and taste abnormalities may be induced. Xerostomia may also affect the development of oral diseases such as oral ulcers. Xerostomia may be diagnosed by sialometry (an unstimulated saliva flow rate of 0.1 mL/min or less), sialography, biopsy, or nuclear medicine examination. Xerostomia may also be diagnosed through salivary gland biopsy, and may be diagnosed based on the degree of parenchymal atrophy of salivary gland in the salivary gland tissue. Sjogren's syndrome may be divided according to scores from 0 to 6 based on lymphocytic infiltration levels by measuring the lymphocytic infiltration patterns in the salivary gland tissue as an evaluation of salivary gland hypofunction.

The composition may be for detection in a saliva sample.

The expression level of at least one gene, protein or fragment thereof selected from the group consisting of NUCB2, PDCD6IP, PTGR1, FAM3D, and ZG16B may be decreased, as compared with that of a normal control group.

NUCB2 is a calcium-binding protein which may have a role in calcium homeostasis, and may be also called HEL-S-109 or NEFA. NUCB2 protein may be a protein encoded by a NUCB2 gene. NUCB2 may include an amino acid sequence of UniProt Accession No. P80303 in human. NUCB2 may include an amino acid sequence of UniProt Accession No. P81117 in mouse.

PDCD6IP is a protein which has a role in endocytosis or programmed cell death, and may be also called AIP1, ALIX, DRIP4, or HP95. Overexpression of PDCD6IP may block apoptosis. PDCD6IP may include an amino acid sequence of UniProt Accession No. Q8WUM4 in human. PDCD6IP may include an amino acid sequence of UniProt Accession No. Q9WU78 in mouse.

PTGR1 is a NAD(P)H-dependent oxidoreductase involved in metabolic inactivation of inflammatory and anti-inflammatory eicosanoids of prostaglandin, leukotriene and lipoxin. PTGR1 may be also called PRG-1, 15-oxoprostaglandin 13-reductase, dithiolethione-inducible gene 1 protein, DIG-1, leukotriene B4 12-hydroxydehydrogenase (LTB4DH), or NAD(P)H-dependent alkenal/one oxidoreductase. PTGR1 may include an amino acid sequence of UniProt Accession No. Q14914 in human. PTGR1 may include an amino acid sequence of UniProt Accession No. P48758 in mouse.

FAM3D may have a role in cytokine activity and may be responsible for negative regulation of glucagon secretion or insulin secretion. FAM3D may include an amino acid sequence of UniProt Accession No. Q96BQ1 in human. FAM3D may include an amino acid sequence of UniProt Accession No. P97805 in mouse.

ZG16B may have a role in carbohydrate binding or retinal homeostasis. ZG16B may be also called EECP, HRPE773, JCLN2, PAUF, PRO1567, or LOC124220. ZG16B may include an amino acid sequence of UniProt Accession No. Q96DA0 in human. ZG16B may include an amino acid sequence of UniProt Accession No. A0A0G2K7Y9 in rat.

The expression level of at least one gene, protein or fragment thereof selected from the group consisting of A1AT, ANT3, ATPA, HRG, H15, H2B1L, IGG1, PCBP1, and PTPRJ may be increased, as compared with that of a normal control group.

A1AT is a protein belonging to the serpin superfamily. A1AT may be also called SERPINA1, A1A, AAT, PI, PI1, PRO2275, alpha1AT, serpin family A member 1, or nNIF. A1AT may be a protease inhibitor that inhibits various proteases. A1AT may be a single-chain glycoprotein. A1AT may include an amino acid sequence of UniProt Accession No. P01009 in human.

ANT3 is a small protein that inactivates several enzymes in the blood coagulation system. ANT3 may inactivate thrombin in plasma. ANT3 may be also called SERPINC1, AT3, AT3D, ATIII, THPH7, serpin family C member 1, ATIII-R2, ATIII-T2, or ATIII-T1. ANT3 may include an amino acid sequence of UniProt Accession No. P01008 in human. ANT3 may include an amino acid sequence of UniProt Accession No. P32261 in mouse.

ATPA is a subunit constituting a protein that biosynthesizes ATP from ADP under a proton concentration gradient generated by the electron transport complex during cellular respiration. ATPA may be also called ATP synthase F1 subunit alpha. ATPA may include an amino acid sequence of UniProt Accession No. P25705 in human. ATPA may include an amino acid sequence of UniProt Accession No. Q03265 in mouse.

HRG is a glycoprotein produced in the liver. HRG may be also called HPRG, HRGP, or THPH11. HRG may include an amino acid sequence of UniProt Accession No. P04196 in human. HRG may include an amino acid sequence of UniProt Accession No. Q9ESB3 in mouse.

H15 is a protein that binds to a linker DNA between nucleosomes to form a macromolecular structure called chromatin. H15 may be also called Histone H1a, Histone H1b, or Histone H1s-3. H15 may include an amino acid sequence of UniProt Accession No. P16401 in human. H15 may include an amino acid sequence of UniProt Accession No. P43276 in mouse.

H2B1L is one of proteins constituting nucleosomes. H2B1L may be also called Histone H2B.c. H2B1L may include an amino acid sequence of UniProt Accession No. Q99880 in human.

IGG1 may be a heavy chain of an antibody. IGG1 may be also called IGHG1 or Immunoglobulin gamma-1 heavy chain NIE. IGG1 may include an amino acid sequence of UniProt Accession No. P0DOX5 in human. IGG1 may include an amino acid sequence of UniProt Accession No. P01868 in mouse.

PCBP1 is a single-stranded nucleic acid binding-protein that binds to oligo dC. PCBP1 may be also called HEL-S-85, HNRPE1, HNRPX, hnRNP-E1 or hnRNP-X. PCBP1 may include an amino acid sequence of UniProt Accession No. Q15365 in human. PCBP1 may include an amino acid sequence of UniProt Accession No. P60335 in mouse.

PTPRJ is a tyrosine phosphatase involved in dephosphorylation of CTNND1, FLT3, PDGFRB, MET, RET (variant MEN2A), KDR, LYN, SRC, MAPK1, MAPK3, EGFR, TJP1, OCLN, PIK3R1, and PIK3R2. PTPRJ may be also called CD148, DEP1, HPTPeta, R-PTP-ETA or SCC1. PTPRJ may include an amino acid sequence of UniProt Accession No. Q12913 in human. PTPRJ may include an amino acid sequence of UniProt Accession No. Q64455 in mouse.

The fragment is a part of the protein and may be an immunogenic polypeptide.

The gene refers to a nucleic acid encoding the protein. The expression level of the gene may be an expression level of mRNA encoding the protein. The expression level of mRNA may be a relative or absolute amount of mRNA. The measuring of the expression level of the gene may be measuring of the amount of mRNA.

The expression level of the protein or fragment thereof may be a relative or absolute amount of the protein or fragment thereof. The measuring of the expression level of the protein or fragment thereof may be measuring of the amount of the protein or fragment thereof.

The agent may be an antibody or an antigen-binding fragment thereof or an aptamer that specifically binds to the protein or fragment thereof. The antibody may be a polyclonal antibody or a monoclonal antibody. The term "antibody" may be used interchangeably with the term "immunoglobulin". The antibody may be a polyclonal antibody or a monoclonal antibody. The antibody may be a full-length antibody. The antigen-binding fragment refers to a polypeptide including an antigen-binding site. The antigen-binding fragment may be a single-domain antibody, Fab, Fab', or scFv. The antibody or antigen-binding fragment may be attached to a solid support. The solid support is, for example, the surface of a metal chip, plate, or well. The antibody or antigen-binding fragment may be an antibody or antigen-binding fragment. The aptamer may be a single-stranded nucleic acid (DNA, RNA, or modified nucleic acid) or a peptide that specifically binds to the protein or fragment thereof.

The agent may be a nucleic acid including a polynucleotide identical to or complementary to the polynucleotide encoding the protein or fragment thereof. The nucleic acid may be a primer, a probe, or an antisense oligonucleotide. The primer, probe, or antisense oligonucleotide may be labeled with a fluorescent material, a chemiluminescent material, or a radioactive isotope at the end or inside thereof.

The composition may further include an agent for measuring an expression level of at least one gene, protein or fragment thereof selected from the group consisting of immunoglobulin kappa variable 1-8 (KV108), Golgi apparatus protein 1 (GSLG1), protein FAM3B (FAM3B), mucin-21 (MUC21), Ras-related protein Rab-11A (RB11A), lysozyme C (LYSC), calmodulin-like protein 3 (CALL3), alpha-1B-glycoprotein (A1BG), alpha-1-acid glycoprotein 1 (A1AG1), calreticulin (CALR), plasminogen (PLMN), extracellular matrix protein 1 (ECM1), annexin A6 (ANXA6), WD repeat-containing protein 1 (WDR1), leukocyte elastase inhibitor (ILEU), alpha-2-HS-glycoprotein (FETUA), myeloid cell nuclear differentiation antigen (MNDA), matrix metalloproteinase-9 (MMP9), neutrophil gelatinase-associated lipocalin (NGAL), D-dopachrome decarboxylase (DOPD), and neutrophil collagenase (MMP8).

The expression level of at least one gene, protein or fragment thereof selected from the group consisting of KV108, GSLG1, FAM3B, MUC21, RB11A, LYSC, and CALL3 may be decreased, as compared with that of a normal control group.

KV108 may be a variable region of an immunoglobulin light chain. KV108 may be also called IGKV18, L9, or IGKV1-8. KV108 may include an amino acid sequence of UniProt Accession No. A0A0C4DH67 in human.

GSLG1 may bind to fibroblast growth factor and E-selectin. GSLG1 may be also called cysteine-rich fibroblast growth factor receptor (CFR-1), E-selectin ligand 1 (ESL-1), or Golgi sialoglycoprotein MG-160. GSLG1 may include an amino acid sequence of UniProt Accession No. Q92896 in human. GSLG1 may include an amino acid sequence of UniProt Accession No. Q61543 in mouse.

FAM3B may induce apoptosis in alpha and beta cells in the pancreas. FAM3B may be also called cytokine-like protein 2-21 or Pancreatic-derived factor (PANDER). FAM3B may include an amino acid sequence of UniProt Accession No. P58499 in human. FAM3B may include an amino acid sequence of UniProt Accession No. Q9D309 in mouse.

MUC21 may be a biomarker for lung carcinoma. MUC21 may be also called Epiglycanin. MUC21 may include an amino acid sequence of UniProt Accession No. Q5SSG8 in human. MUC21 may include an amino acid sequence of UniProt Accession No. D9N008 or A8QW50 in mouse.

RB11A may be a small GTPase Rab that regulates intracellular membrane migration. MUC21 may be also called Rab-11 or YL8. RB11A may include an amino acid sequence of UniProt Accession No. P62491 in human. RB11A may include an amino acid sequence of UniProt Accession No. P62492 in mouse.

LYSC is an enzyme that has a role in hydrolysis and glycosylation. LYSC may be also called 1,4-beta-N-acetylmuramidase C. LYSC may include an amino acid sequence of UniProt Accession No. P61626 in human. LYSC may include an amino acid sequence of UniProt Accession No. P17897 or P08905 in mouse.

CALL3 may function as a specific light chain of myosin-10. CALL3 may be also called CaM-like protein (CLP) or calmodulin-related protein NB-1. CALL3 may include an amino acid sequence of UniProt Accession No. P27482 in human. CALL3 may include an amino acid sequence of UniProt Accession No. Q9D6P8 in mouse.

The expression level of at least one gene, protein or fragment thereof selected from the group consisting of A1BG, A1AG1, CALR, PLMN, ECM1, ANXA6, WDR1, ILEU, FETUA, MNDA, MMP9, NGAL, DOPD, and MMP8 may be increased, as compared with that of a normal control group.

A1BG may have a role in degranulation of neutrophils or platelets. A1BG may be also called A1B, ABG, GAB, HYST2477, or alpha-1-B glycoprotein. A1BG may include an amino acid sequence of UniProt Accession No. P04217 in human. A1BG may include an amino acid sequence of UniProt Accession No. Q19LI2 in mouse.

A1AG1 may function as a transfer protein in the blood. A1AG1 may be also called AGP 1 or Orosomucoid-1 (OMD 1). A1AG1 may include an amino acid sequence of UniProt Accession No. P02763 in human. A1AG1 may include an amino acid sequence of UniProt Accession No. Q60590 in mouse.

CALR is a water-soluble protein that binds to calcium ions. CALR may be also called CRT, HEL-S-99n, RO, SSA, or cC1qR. CALR may include an amino acid sequence of UniProt Accession No. P27797 in human. CALR may include an amino acid sequence of UniProt Accession No. P14211 in mouse.

PLMN is a precursor of plasmin. PLMN may be cleaved into plasmin heavy chain A, activation peptide, angiostatin, plasmin heavy chain A (short form), and plasmin light chain B. PLMN may include an amino acid sequence of UniProt Accession No. P00747 in human. PLMN may include an amino acid sequence of UniProt Accession No. P06869 in mouse.

ECM1 is a negative regulator of bone mineralization and is involved in endochondral bone formation. ECM1 may be also called secretory component p85. ECM1 may include an amino acid sequence of UniProt Accession No. Q16610 in human. ECM1 may include an amino acid sequence of UniProt Accession No. Q61508 in mouse.

ANXA6 is a calcium-dependent membrane and phospholipid binding protein. ANXA6 may be also called ANX6, CBP68, annexin A6, CPB-II, p70, or p68. ANXA6 may include an amino acid sequence of UniProt Accession No. P08133 in human. ANXA6 may include an amino acid sequence of UniProt Accession No. P14824 in mouse.

WDR1 may be involved in protein-protein interactions. WDR1 may be also called AIP1, HEL-S-52, or NORI-1. WDR1 may include an amino acid sequence of UniProt Accession No. 075083 in human. WDR1 may include an amino acid sequence of UniProt Accession No. 088342 in mouse.

ILEU is a neutrophil serine protease inhibitor involved in regulation of the innate immune response, inflammation, and cellular homeostasis. ILEU may be also called LEI, El, monocyte/neutrophil elastase inhibitor (M/NEI), peptidase inhibitor 2 (PI-2), or Serpin B1. ILEU may include an amino acid sequence of UniProt Accession No. P30740 in human. ILEU may include an amino acid sequence of UniProt Accession No. Q9D154 in mouse.

FETUA is a plasma-binding protein abundant in the fetus. FETUA may be also called alpha-2-HS-glycoprotein (AHSG), A2HS, AHS, or HSGA. FETUA may include an amino acid sequence of UniProt Accession No. P02765 in human. FETUA may include an amino acid sequence of UniProt Accession No. P29699 in mouse.

MNDA is a protein detected in nuclei of cells of the granulocyte-monocyte lineage. FETUA may be also called PYHIN3. MNDA may include an amino acid sequence of UniProt Accession No. P41218 in human.

MMP9 is one of zinc-metalloproteinases involved in degradation of the extracellular matrix. MMP9 may be also called CLG4B, GELB, MANDP2, MMP-9, 92 kDa type IV collagenase, 92 kDa gelatinase, or gelatinase B. MMP9 may include an amino acid sequence of UniProt Accession No. P14780 in human. MMP9 may include an amino acid sequence of UniProt Accession No. P41245 in mouse.

NGAL is a protein involved in innate immunity. NGAL may be also called LCN2, 24p3, MSFI, p25, or lipocalin 2. NGAL may include an amino acid sequence of UniProt Accession No. P80188 in human. NGAL may include an amino acid sequence of UniProt Accession No. P11672 in mouse.

DOPD is an enzyme that decarboxylates D-dopachrome to 5,6-dihydroxyindole (DHI). DOPD may be also called phenylpyruvate tautomerase II. DOPD may include an amino acid sequence of UniProt Accession No. P30046 in human.

MMP8 is an enzyme capable of degrading fibrous type I, II, and III collagen. MMP8 may be also called Matrix metalloproteinase-8 (MMP-8) or PMNL-CL. MMP8 may include an amino acid sequence of UniProt Accession No. P22894 in human. MMP8 may include an amino acid sequence of UniProt Accession No. 070138 in mouse.

Another aspect provides use of a kit for diagnosing xerostomia, the kit including an agent for measuring an expression level of at least one gene, protein or fragment thereof selected from the group consisting of NUCB2, PDCD6IP, PTGR1, FAM3D, ZG16B, A1AT, ANT3, ATPA, HRG, H15, H2B1L, IGG1, PCBP1, and PTPRJ, or an expression level of a protein or fragment thereof.

NUCB2, PDCD6IP, PTGR1, FAM3D, ZG16B, A1AT, ANT3, ATPA, HRG, H15, H2B1L, IGG1, PCBP1, PTPRJ, and xerostomia are the same as described above.

The kit may further include a sample needed for diagnosing xerostomia. The kit may include a solid support, a substrate for immunological detection of an antibody or antigen-binding fragment, a suitable buffer, a chromogenic enzyme, a secondary antibody labeled with a fluorescent material, or a chromogenic substrate. The kit may include a polymerase, a buffer, a nucleic acid, a coenzyme, a fluorescent material, or a combination thereof for nucleic acid detection. The polymerase is, for example, Taq polymerase.

The kit may further include an agent for measuring an expression level of at least one gene, protein or fragment thereof selected from the group consisting of KV108, GSLG1, FAM3B, MUC21, RB11A, LYSC, CALL3, A1BG, A1AG1, CALR, PLMN, ECM1, ANXA6, WDR1, ILEU, FETUA, MNDA, MMP9, NGAL, DOPD, and MMP8.

Still another aspect provides a method for diagnosing xerostomia, the method including measuring an expression level of at least one gene, protein or fragment thereof wherein the at least one gene, protein or fragment thereof is NUCB2, in a saliva sample isolated from a subject suspected of having xerostomia; comparing the measured expression level with an expression level of a normal control group; and determining the subject as having xerostomia, when the expression level of the least one gene, protein or fragment thereof of NUCB2, is decreased, as compared with that of the normal control group,

NUCB2, PDCD6IP, PTGR1, FAM3D, ZG16B, A1AT, ANT3, ATPA, HRG, H15, H2B1L, IGG1, PCBP1, PTPRJ, and xerostomia are the same as described above.

The method includes further measuring an expression level of at least one gene, protein or fragment thereof selected from the group consisting of immunoglobulin kappa variable 1-8 (KV108), Golgi apparatus protein 1 (GSLG1), protein FAM3B (FAM3B), mucin-21 (MUC21), Ras-related protein Rab-11A (RB11A), lysozyme C (LYSC), calmodulin-like protein 3 (CALL3), alpha-1B- glycoprotein (A1BG), alpha-1-acid glycoprotein 1 (A1AG1), calreticulin (CALR), plasminogen (PLMN), extracellular matrix protein 1 (ECM1), annexin A6 (ANXA6), WD repeat-containing protein 1 (WDR1), leukocyte elastase inhibitor (ILEU), alpha-2-HS- glycoprotein (FETUA), myeloid cell nuclear differentiation antigen (MNDA), matrix metalloproteinase-9 (MMP9), neutrophil gelatinase-associated lipocalin (NGAL), D-dopachrome decarboxylase (DOPD), neutrophil collagenase (MMP8), programmed cell death 6-interacting protein (PDCD6IP), prostaglandin reductase 1 (PTGR1), FAM3D, Zymogen granule protein 16 homolog B (ZG16B), alpha-1-antitrypsin (A1AT), antithrombin-III (ANT3), ATP synthase subunit alpha (ATPA, mitochondrial), histidine- rich glycoprotein (HRG), histone H1.5 (H15), histone H2B type 1-L (H2B1L), immunoglobulin gamma-1 heavy chain (IGG1), poly(rC)-binding protein 1 (PCBP1), and receptor-type tyrosine-protein phosphatase eta (PTPRJ). a saliva sample isolated from a subject suspected of having xerostomia.

The subject may be a mammal, for example, a human, a cow, a horse, a pig, a dog, a sheep, a goat, or a cat. The subject may be a subject suffering from xerostomia disorder or suspected of having xerostomia.

The saliva sample refers to a saliva sample obtained from the subject. The saliva sample may be a sample that has been subjected to pretreatment such as centrifugation, filtration, etc. The saliva sample includes a protein sample or a nucleic acid sample obtained from the saliva sample.

The measuring may include incubating the saliva sample with an antibody or antigen-binding fragment thereof, or an aptamer specifically binding to the protein or fragment thereof, or with a polynucleotide identical or complementary to the polynucleotide encoding the gene.

The measuring may be performed by electrophoresis, immunoblotting, enzyme-linked immunosorbent assay (ELISA), Northern blotting, polymerase chain reaction (PCR), a protein chip, immunoprecipitation, a microarray, an electron microscopy, or a combination thereof. The electrophoresis may be SDS-PAGE, isoelectric point electrophoresis, two-dimensional electrophoresis, or a combination thereof.

The method includes comparing the measured expression level with an expression level of a normal control group.

The method may include determining whether an expression level of at least one gene, protein or fragment thereof selected from the group consisting of NUCB2, PDCD6IP, PTGR1, FAM3D, and ZG16B is decreased, as compared with that of a normal control group. The normal control group, which is a group without xerostomia, refers to a negative control group. In the method, the expression level of at least one gene, protein or fragment thereof selected from the group consisting of NUCB2, PDCD6IP, PTGR1, FAM3D, and ZG16B measured in the saliva sample may be decreased, as compared with that of the normal control group, for example, when the detected amount of the biomarker is lower than that of the normal control group, the subject may be diagnosed as having xerostomia or having a high probability of xerostomia.

The method may include confirming whether an expression level of at least one gene, protein or fragment thereof selected from the group consisting of A1AT, ANT3, ATPA, HRG, H15, H2B1L, IGG1, PCBP1, and PTPRJ is increased, as compared with that of a normal control group. The normal control group, which is a group without xerostomia, refers to a negative control group. In the above method, the measured expression level of at least one gene, protein or fragment thereof selected from the group consisting of A1AT, ANT3, ATPA, HRG, H15, H2B1L, IGG1, PCBP1, and PTPRJ in the saliva sample may be increased, as compared with that of the normal control group, for example, when the detected amount of the biomarker is larger than that of the normal control group, the subject may be diagnosed as having xerostomia or having a high probability of xerostomia.

Subjects diagnosed as xerostomia may be treated with oral care, control of drug administration, administration of saliva substitutes, administration of oral moisturizer, electrical stimulation or acupuncture to the oral cavity.

The method may further include measuring an expression level of at least one gene, protein or fragment thereof selected from the group consisting of KV108, GSLG1, FAM3B, MUC21, RB11A, LYSC, CALL3, A1BG, A1AG1, CALR, PLMN, ECM1, ANXA6, WDR1, ILEU, FETUA, MNDA, MMP9, NGAL, DOPD, and MMP8.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 shows SDS-PAGE gel images of saliva samples of a normal control group and xerostomia patient groups;
FIG. 2 shows Venn diagrams showing the number of proteins identified in saliva samples and the number of statistically significant proteins with a p-value of less than 0.05;
FIGS. 3A to 3E show graphs showing expression levels of NUCB2, PDCD6IP, PTGR1, FAM3D, and ZG16B according to scores of normal control groups and xerostomia patient groups (y-axis: LFQ intensity, N: normal control group, S1: score 1, S2: score 2, S345: score 3 or higher); and
FIGS. 4A to 4I show graphs showing expression levels of A1AT, ANT3, ATPA, HRG, H15, H2B1L, IGG1, PCBP1, and PTPRJ according to scores of normal control groups and xerostomia patient groups (y-axis: LFQ intensity, N: normal control group, S1: score 1, S2: score 2, S345: score 3 or higher).

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

Hereinafter, the present disclosure will be described in more detail with reference to exemplary embodiments. However, these exemplary embodiments are only for illustrating one or more specific embodiments, and the scope of the present disclosure is not limited to these exemplary embodiment.

### Example 1. Identification of Biomarkers Differentially Expressed in Saliva Samples of Xerostomia Patients

### 1. Preparation of saliva samples of xerostomia patients

Saliva samples were collected from normal control groups (n=3) and patients diagnosed with xerostomia from Hanyang University Hospital. Xerostomia was diagnosed by clinical examination of salivary secretion and oral mucosa condition and by a test of salivary gland function through salivary gland scans. Salivary gland function was classified into score 1 to score 5 according to the condition of the salivary gland tissue and lymphocytic infiltration around the salivary gland parenchyma through a salivary gland biopsy. Saliva sampling was performed by classifying into three groups of score 1, score 2, and scores 3 to 5.

To prevent protein degradation or modification of the saliva sample, cOmplete as a protease inhibitor, Mini EDTA-free protease inhibitor cocktail (Roche), and PhosSTOP (Roche) as a phosphatase inhibitor were added to the collected saliva samples immediately after collecting sera. After quantifying the saliva sample to which the inhibitors were added, it was stored at -80°C until use.

### 2. Concentration and quantification of saliva samples

A total of 12 saliva samples from 4 groups were concentrated using a 3k centrifugal filter (Amicon Ultra, Millipore). A filter was prepared by adding, to the 3k centrifugal filter, 400 µl of water (HPLC grade, J.T Baker) twice and 400 µl of 10 mM Tris buffer (pH 8.0) containing 0.1% (w/v) SDS twice.

Each saliva sample was diluted 2-fold with 10 mM Tris buffer containing 0.1% (w/v) SDS, followed by vortexing to remove precipitates. Each diluted sample was applied to the prepared filter, and then the sample was concentrated by centrifugation at a speed of 14000x g at 10°C for about 10 minutes. Thereafter, the filter containing the sample was washed five times with 10 mM Tris buffer containing 0.05% (w/v) SDS. After inserting the filter containing the sample concentrated after washing upside down into a new tube, centrifugation was performed at a speed of 3000x g at 10°C for about 2 minutes to recover the concentrated sample.

The recovered sample was quantified by a bicinchoninic acid (BCA) assay.

### 3. Preparation of samples for mass spectrometry and in-gel digestion

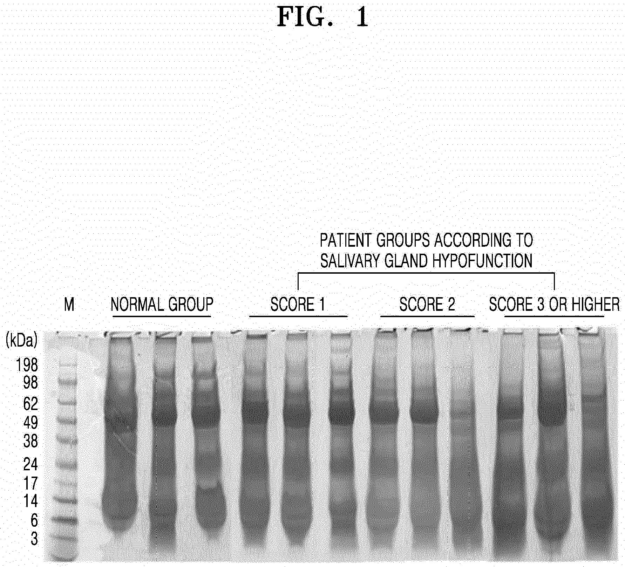

For proteomic analysis, each 55 µg of 12 individual samples from 4 groups obtained in 2. was separated according to molecular weights using NuPAGE gels (Bis-Tris gels from 4% to 12%, Invitrogen, Carlsbad, CA, USA). The gel was stained with Coomassie Brilliant Blue R-250 (FIG. 1). Each stained gel for 12 individual samples was divided into 12 slices, and each gel slice was transferred to a microcentrifuge tube. Disulfide bonds of the proteins contained in each gel slice were reduced at 56 °C, alkylated in a dark environment at 25 °C, and digested with trypsin overnight. Then, the peptides were extracted using a solution of 67% (v/v) acetonitrile (ACN)/5% (v/v) formic acid (FA) (Wako Pure Chemicals, Osaka, Japan). The extracted peptides were dried in Speedvac and dissolved in 20 µl of 0.4% (v/v) acetic acid.

### 4. Mass spectrometry analysis

10 µl of each sample obtained in 3. resuspened in 0.4% acetic acid was injected into a reversed-phase Magic C18AQ column (15 cm X 75 µm) on an Eksigent MDLC system (Eksigent Technologies Dublin, CA, USA). The column was equilibrated with a buffer prepared by mixing 95% buffer A (100% (v/v) water containing 0.1% (v/v) formic acid) and 5% buffer B (100% (v/v) ACN containing 0.1% (v/v) formic acid). The working flow rate was 0.35 µl/min under the following gradient conditions:

0-8% buffer B at 0 min to 5 min; 8-30% buffer B at 5 min to 85 min; 30-70% buffer B at 85 min to 90 min; 70% buffer B at 90 min to 95 min; 70-2% buffer B at 95 min to 100 min; 2% buffer B at 100 min to 110 min.

A nano HPLC system was mounted on an LTQ XL-Orbitrap mass spectrometer (Thermo Scientific, San Jose, CA, USA). A spray voltage was set at 2.5 kV, and a temperature of a heated capillary was set at 250 °C. Survey full-scan MS spectra (300 m/z to 1800 m/z) were acquired with 1 microscan and a resolution of 60,000 allowing the preview mode for precursor selection and charge-state determination. MS/MS spectra of ten most intense ions from the preview survey scan were acquired in the ion-trap concurrent with full-scan with the following options: isolation width, 2 m/z (mass to charge); normalized collision energy, 35%; dynamic exclusion duration, 360 seconds. Precursors with +1 charge and unmatched charge state were discarded during data dependent acquisition.

Each LC-MS/MS file was analyzed using an Andromeda algorithm of MaxQunat1.5.8.3. MS and MS/MS data were compared with SwissProt human database (January 2019). The investigation was limited to accepting tryptic digested peptides with two missed cleavages. Carbamidomethylation of cysteine (+57.021 Da) was set as fixed modifications and methionine oxidation (+15.995 Da) as variable modifications. Mass tolerance was set at 0.5 Da for MS/MS data and 15 ppm for MS data. For identification from decoy database, a total of two target values were applied: a false discovery rate (FDR) of 0.01 and a relaxed FDR of 0.05. All proteins were identified using two or more matching peptides.

### 5. Label-free quantification analysis

Based on label-free quantification intensity (LFQ intensity), the relative abundance of proteins present in a total of 12 samples obtained by collecting three saliva samples each from a normal control group and three groups of xerostomia patient groups was calculated. The LFQ intensity was extracted using MaxQunat1.5.8.3. Subsequently, LFQ intensity for each protein was measured for relative quantitative analysis using Perseus. Even when the protein was absent or below the detection limit, the null value was compensated. In order to calculate a fold difference between the normal control groups and the xerostomia patient groups, missing values were imputed from a normal distribution with a width of 0.3 and a downshift of 1.8. Statistical significance was performed on the LFQ intensity obtained from an ANOVA test with three individual samples of the normal control groups and the xerostomia patient groups, and a p-value of less than 0.05 was considered statistically significant.

### 6. Identification of salivary proteins associated with xerostomia patient groups and discovery of differentially expressed proteins:

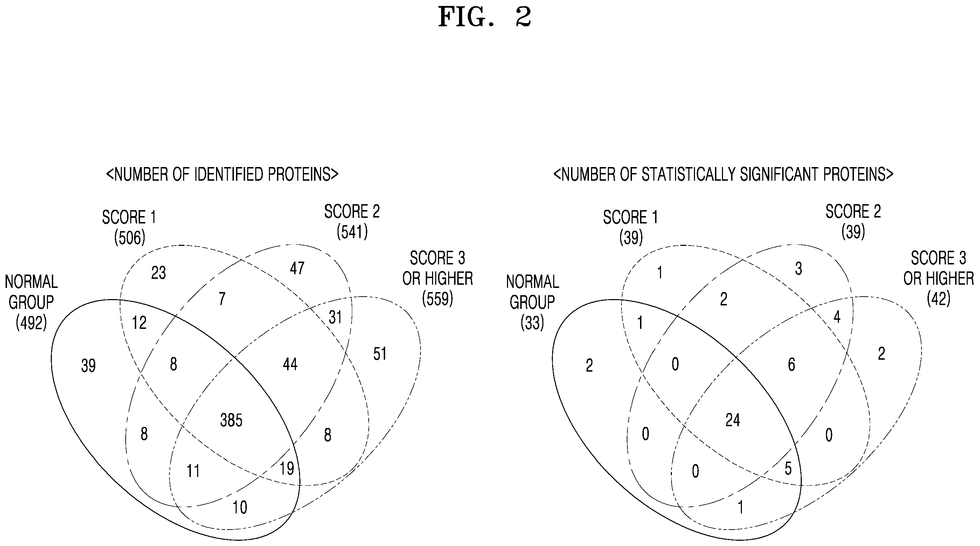

From the LFQ intensity analysis, a total of 703 proteins were identified in 12 individual samples of the normal control groups and xerostomia groups. Among the identified proteins, 51 proteins with a p-value of less than 0.05 were identified. Venn diagrams showing the results of the number of identified proteins and the number of statistically significant proteins with a p-value of less than 0.05 are shown in FIG. 2.

In addition, the statistically significant proteins with a p-value of less than 0.05 are shown in Table 1.

**[Table 1]**

| UniProt Accession No. | Gene name | Protein name | p-value |
|---|---|---|---|
| P04217 | A1BG | Alpha-1B-glycoprotein | 8.1694E-07 |
| P02763 | A1AG1 | Alpha-1-acid glycoprotein 1 | 0.0004 |
| P27797 | CALR | Calreticulin | 0.001 |
| P40199 | CEAM6 | Carcinoembryonic antigen-related cell adhesion molecule 6 | 0.002 |
| A0A0C4DH6 7 | KV108 | Immunoglobulin kappa variable 1-8 | 0.002 |
| P01008 | ANT3 | Antithrombin-Ill | 0.004 |
| Q8TAX7 | MUC7 | Mucin-7 | 0.005 |
| Q16778 | H2B2E | Histone H2B type 2-E | 0.005 |
| P04196 | HRG | Histidine-rich glycoprotein | 0.005 |
| Q14914 | PTGR1 | Prostaglandin reductase 1 | 0.007 |
| P02751 | FINC | Fibronectin | 0.007 |
| 075131 | CPNE3 | Copine-3 | 0.008 |
| P0DOX5 | IGG1 | Immunoglobulin gamma-1 heavy chain | 0.009 |
| P60842 | IF4A1 | Eukaryotic initiation factor 4A-I | 0.009 |
| P25705 | ATPA | ATP synthase subunit alpha, mitochondrial | 0.009 |
| Q92896 | GSLG1 | Golgi apparatus protein 1 | 0.012 |
| P00747 | PLMN | Plasminogen | 0.014 |
| P58499 | FAM3B | Protein FAM3B | 0.016 |
| Q16610 | ECM1 | Extracellular matrix protein 1 | 0.016 |
| P08133 | ANXA6 | Annexin A6 | 0.016 |
| 015511 | ARPC5 | Actin-related protein 2/3 complex subunit 5 | 0.018 |
| P01009 | A1AT | Alpha-1-antitrypsin | 0.020 |
| Q05315 | LEG10 | Galectin-10 | 0.021 |
| A0A0A0MS1 5 | HV349 | Immunoglobulin heavy variable 3-49 | 0.022 |
| Q96DA0 | ZG16B | Zymogen granule protein 16 homolog B | 0.025 |
| P20591 | MX1 | Interferon-induced GTP-binding protein Mx1 | 0.025 |
| 075083 | WDR1 | WD repeat-containing protein 1 | 0.026 |
| P30740 | ILEU | Leukocyte elastase inhibitor | 0.028 |
| Q99880 | H2B1L | Histone H2B type 1-L | 0.028 |
| Q5SSG8 | MUC21 | Mucin-21 | 0.030 |
| P02765 | FETUA | Alpha-2-HS-glycoprotein | 0.032 |
| Q12792 | TWF1 | Twinfilin-1 | 0.033 |
| P62491 | RB11A | Ras-related protein Rab-11A | 0.035 |
| P13473 | LAMP2 | Lysosome-associated membrane glycoprotein 2 | 0.036 |
| Q8WUM4 | PDC6I | Programmed cell death 6-interacting protein | 0.037 |
| P62244 | RS15A | 40S ribosomal protein S15a | 0.038 |
| A0A0C4DH3 3 | HV124 | Immunoglobulin heavy variable 1-24 | 0.039 |
| Q12913 | PTPRJ | Receptor-type tyrosine-protein phosphatase eta | 0.039 |
| P41218 | MNDA | Myeloid cell nuclear differentiation antigen | 0.042 |
| P14780 | MMP9 | Matrix metalloproteinase-9 | 0.042 |
| P80188 | NGAL | Neutrophil gelatinase-associated lipocalin | 0.042 |
| P80303 | NUCB2 | Nucleobindin-2 | 0.043 |
| P30046 | DOPD | D-dopachrome decarboxylase | 0.043 |
| Q9BWD1 | THIC | Acetyl-CoA acetyltransferase, cytosolic | 0.043 |
| Q92876 | KLK6 | Kallikrein-6 | 0.043 |
| P16401 | H15 | Histone H1.5 | 0.043 |
| P61626 | LYSC | Lysozyme C | 0.044 |
| P22894 | MMP8 | Neutrophil collagenase | 0.047 |
| Q96BQ1 | FAM3D | Protein FAM3D | 0.048 |
| Q15365 | PCBP1 | Poly(rC)-binding protein 1 | 0.049 |
| P27482 | CALL3 | Calmodulin-like protein 3 | 0.049 |

Among the statistically significant proteins with a p-value of less than 0.05, proteins, of which expression level decreased as compared with that of the normal control group, are shown in Table 2.

**[Table 2]**

| UniProt Accession No. | Gene name | Protein name | Differential expression ratio (score 1/normal group) | Differential expression ratio (score 2/normal group) | Differential expression ratio (score 3 or higher/nor mal group) |
|---|---|---|---|---|---|
| P80303 | NUCB2 | Nucleobindin-2 | 0.38 | 0.06 | 0.11 |
| Q8WUM4 | PDC6I | Programmed cell death 6-interacting protein | 0.00 (detected only in normal group) | 0.00 (detected only in normal group) | 0.00 (detected only in normal group) |
| Q14914 | PTGR1 | Prostaglandin reductase 1 | 0.00 | 0.00 | 0.13 |
| Q96BQ1 | FAM3D | Protein FAM3D | 0.22 | 0.00 | 0.12 |
| Q96DA0 | ZG16B | Zymogen granule protein 16 homolog B | 0.87 | 0.34 | 0.53 |
| A0A0C4DH6 7 | KV108 | Immunoglobulin kappa variable 1-8 | 0(detected only in normal group) | 0 (detected only in normal group) | 0 (detected only in normal group) |
| Q92896 | GSLG1 | Golgi apparatus protein 1 | 0.46373 | 0 | 0.346157 |
| P58499 | FAM3B | Protein FAM3B | 0.580505 | 0 | 0 |
| Q5SSG8 | MUC21 | Mucin-21 | 0.053745 | 0 | 0.239191 |
| P62491 | RB11A | Ras-related protein Rab-11A | 0.531124 | 0.802295 | 0.512411 |
| P61626 | LYSC | Lysozyme C | 0.577288 | 0.394294 | 0.332109 |
| P27482 | CALL3 | Calmodulin-like protein 3 | 0.433622 | 0.859402 | 0.341424 |

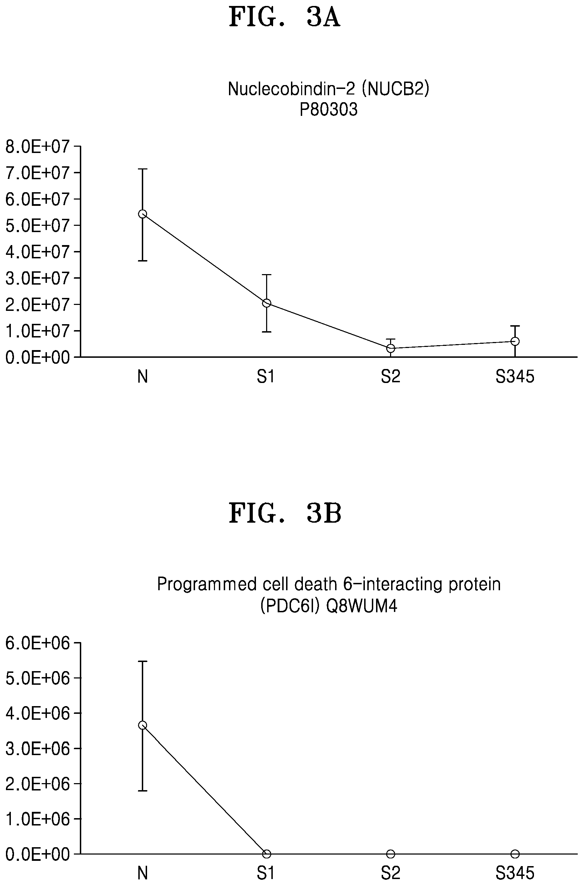

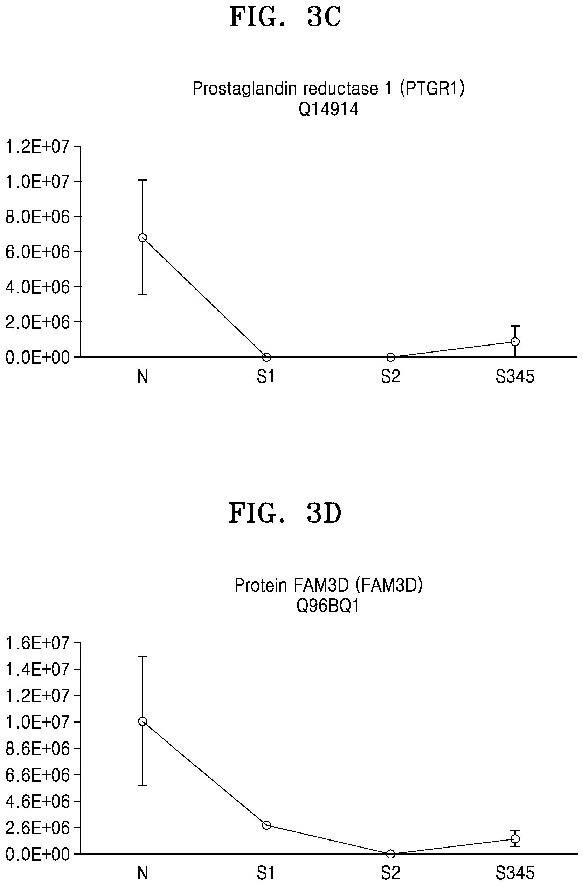

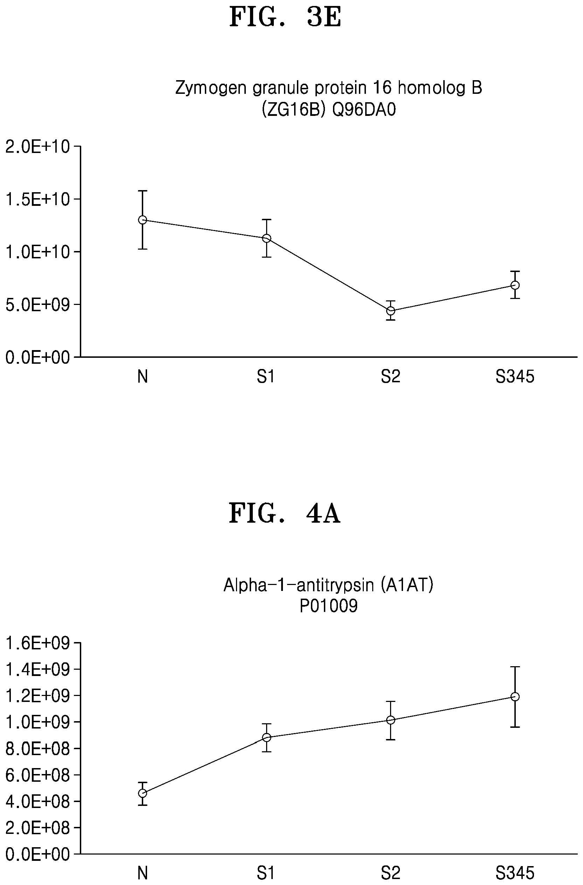

Expression levels of the proteins listed in Table 2 according to the normal control groups and the scores of the xerostomia patient groups are shown in FIGS. 3A to 3E (N: normal control group, S1: score 1, S2: score 2, S345: score 3 or higher).

As shown in FIGS. 3A to 3E, nucleobindin-2 (NUCB2), programmed cell death 6-interacting protein (PDCD6IP), prostaglandin reductase 1 (PTGR1), FAM3D and Zymogen granule protein 16 homolog B (ZG16B) showed significant decreased expression levels in the xerostomia groups, and the expression levels varied according to the xerostomia score.

Next, among the statistically significant proteins with a p-value of less than 0.05, proteins, of which expression level increased 2-fold or more, as compared with that of the normal control group, are shown in Table 3.

**[Table 3]**

| UniProt Accession No. | Gene name | Protein name | Differential expressio n ratio (score 1/normal group) | Differential expression ratio (score 2/normal group) | Differential expression ratio (score 3 or higher/nor mal group) |
|---|---|---|---|---|---|
| P01009 | A1AT | Alpha-1-antitrypsin | 1.92 | 2.21 | 2.60 |
| P01008 | ANT3 | Antithrombin-III | 17.21 | 18.90 | 29.43 |
| P25705 | ATPA | ATP synthase subunit alpha, mitochondrial | 10.39 | 7.36 | 9.77 |
| P04196 | HRG | Histidine-rich glycoprotein | 31.64 | 13.92 | 41.33 |
| P16401 | H15 | Histone H1.5 | 3.83 | 4.51 | 3.71 |
| Q99880 | H2B1L | Histone H2B type 1-L | 2.89 | 3.32 | 2.22 |
| P0DOX5 | IGG1 | Immunoglobulin gamma-1 heavy chain | 2.40 | 3.36 | 2.67 |
| Q15365 | PCBP1 | Poly(rC)-binding protein 1 | 2.00 | 11.17 | 7.70 |
| Q12913 | PTPRJ | Receptor-type tyrosine-protein phosphatase eta | 9.35 | 18.49 | 14.46 |
| P04217 | A1BG | Alpha-1B-glycoprotein | Not detected in normal group | Not detected in normal group | Not detected in normal group |
| P02763 | A1AG1 | Alpha-1-acid glycoprotein 1 | 27.41807 | 40.57206 | 60.38469 |
| P27797 | CALR | Calreticulin | Not detected in normal group | Not detected in normal group | Not detected in normal group |
| P00747 | PLMN | Plasminogen | Not detected in normal group | Not detected in normal group | Not detected in normal group |
| Q16610 | ECM1 | Extracellular matrix protein 1 | Not detected in normal group | Not detected in normal group | Not detected in normal group |
| P08133 | ANXA6 | Annexin A6 | 7.775005 | 7.497202 | 7.419239 |
| 075083 | WDR1 | WD repeat-containing protein 1 | Not detected in normal group | Not detected in normal group | Not detected in normal group |
| P30740 | ILEU | Leukocyte elastase inhibitor | 2.231517 | 6.076515 | 3.099558 |
| P02765 | FETUA | Alpha-2-HS-glycoprotein | Not detected in normal group | Not detected in normal group | Not detected in normal group |
| P41218 | MNDA | Myeloid cell nuclear differentiation antigen | 3.706971 | 4.720786 | 2.538195 |
| P14780 | MMP9 | Matrix metalloproteinase-9 | 2.134616 | 2.763508 | 1.932363 |
| P80188 | NGAL | Neutrophil gelatinase-associated lipocalin | 2.624252 | 2.36209 | 2.40165 |
| P30046 | DOPD | D-dopachrome decarboxylase | Not detected in normal group | Not detected in normal group | Not detected in normal group |
| P22894 | MMP8 | Neutrophil collagenase | 4.38238 | 3.313543 | 3.444907 |

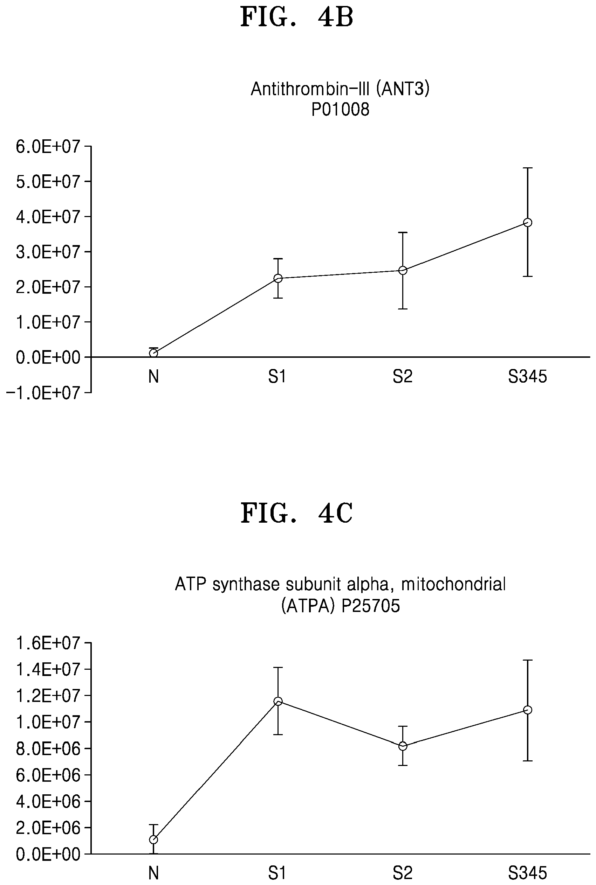

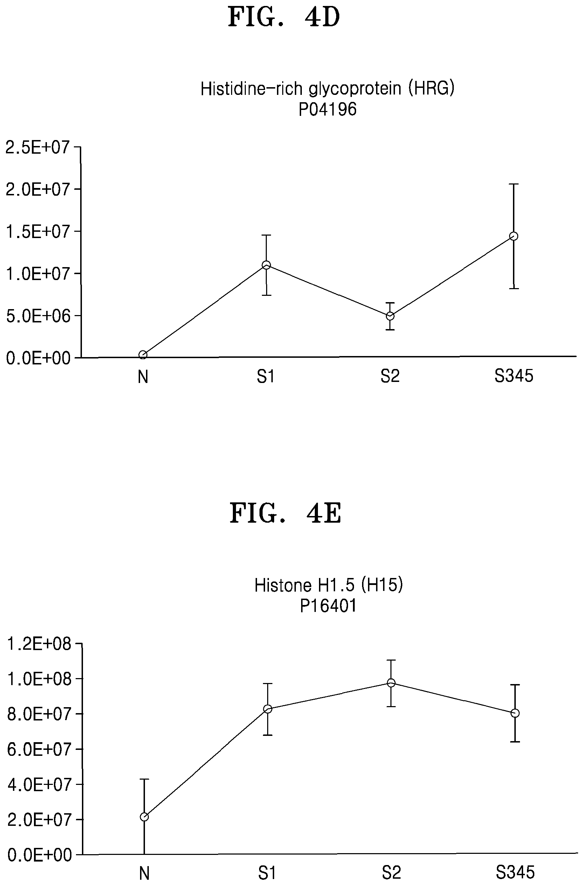

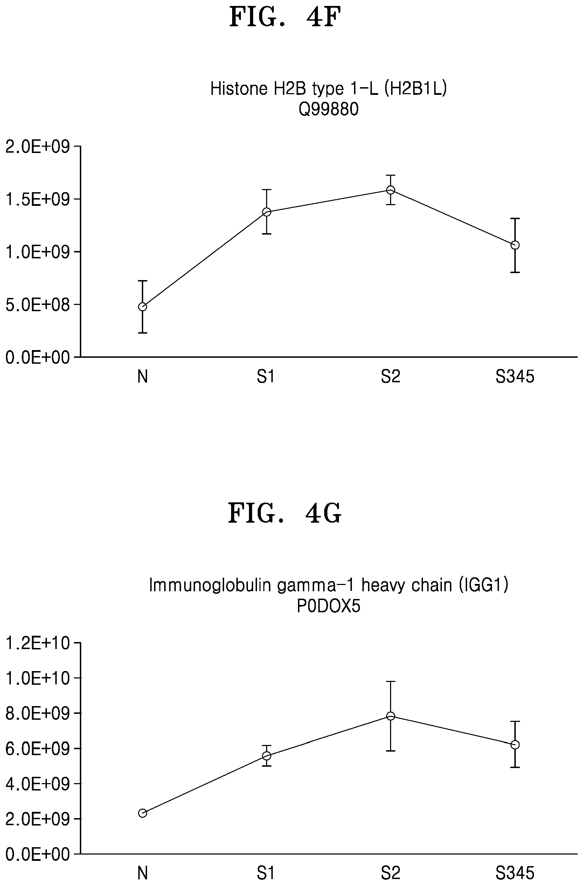

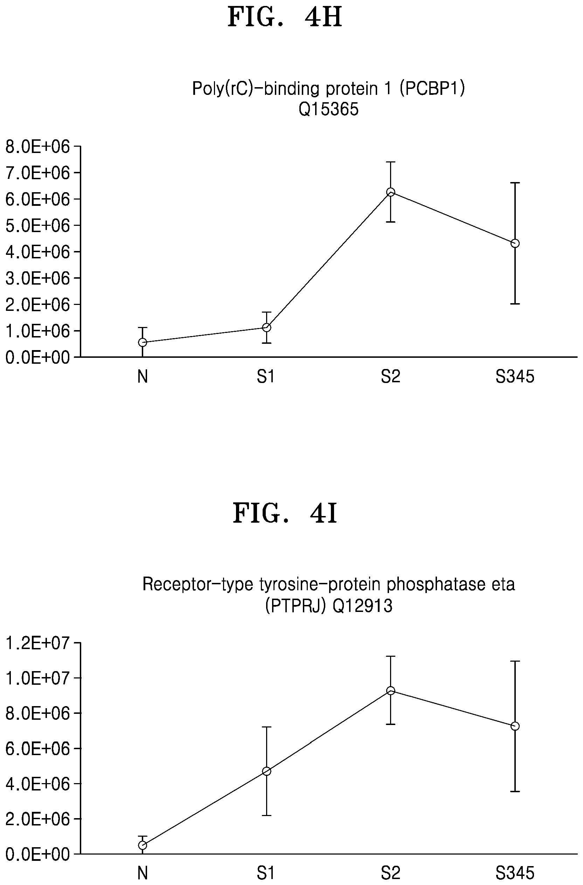

Expression levels of the proteins listed in Table 3 according to the normal control groups and the scores of the xerostomia patient groups are shown in FIGS. 4A to 4I (N: normal control group, S1: score 1, S2: score 2, S345: score 3 or higher).

As shown in FIGS. 4A to 4I, alpha-1-antitrypsin (A1AT), antithrombin-III (ANT3), ATP synthase subunit alpha (ATPA, mitochondrial), histidine-rich glycoprotein (HRG), histone H1.5 (H15), histone H2B type 1-L (H2B1L), immunoglobulin gamma-1 heavy chain (IGG1), poly(rC)-binding protein 1 (PCBP1), and receptor-type tyrosine-protein phosphatase eta (PTPRJ) showed significant increased expression levels in the xerostomia groups, and the expression levels varied according to the xerostomia score.

A composition and a kit, each for diagnosing xerostomia, according to an aspect, and a method of diagnosing xerostomia using the same may be used to diagnose xerostomia simply and objectively with high accuracy and specificity.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the scope of the disclosure as defined by the following claims.

## Claims

1. Use of a composition for diagnosing xerostomia, the composition comprising an agent for measuring an expression level of nucleobindin-2 (NUCB2) gene, protein or fragment thereof, wherein an expression level of NUCB2 gene, protein or fragment thereof is decreased, as compared with that of a normal control group.

2. The use of claim 1, wherein the composition is for detection in a saliva sample.

3. The use of claim 1, wherein the agent is:
an antibody or an antigen-binding fragment thereof or an aptamer that specifically binds to the protein or fragment thereof; or
a nucleic acid comprising a polynucleotide identical to or complementary to a polynucleotide encoding the protein or fragment thereof.

4. The use of claim 3, wherein the antibody is a polyclonal antibody or a monoclonal antibody.

5. The use of claim 3, wherein the nucleic acid is a primer, a probe, or an antisense oligonucleotide.

6. The use of claim 1, further comprising an agent for measuring an expression level of at least one gene, protein or fragment thereof selected from the group consisting of immunoglobulin kappa variable 1-8 (KV108), Golgi apparatus protein 1 (GSLG1), protein FAM3B (FAM3B), mucin-21 (MUC21), Ras-related protein Rab-11A (RB11A), lysozyme C (LYSC), calmodulin-like protein 3 (CALL3), alpha-1B-glycoprotein (A1BG), alpha-1-acid glycoprotein 1 (A1AG1), calreticulin (CALR), plasminogen (PLMN), extracellular matrix protein 1 (ECM1), annexin A6 (ANXA6), WD repeat-containing protein 1 (WDR1), leukocyte elastase inhibitor (ILEU), alpha-2-HS-glycoprotein (FETUA), myeloid cell nuclear differentiation antigen (MNDA), matrix metalloproteinase-9 (MMP9), neutrophil gelatinase-associated lipocalin (NGAL), D-dopachrome decarboxylase (DOPD), neutrophil collagenase (MMP8), programmed cell death 6-interacting protein (PDCD6IP), prostaglandin reductase 1 (PTGR1), FAM3D, Zymogen granule protein 16 homolog B (ZG16B), alpha-1-antitrypsin (A1AT), antithrombin-III (ANT3), ATP synthase subunit alpha (ATPA, mitochondrial), histidine-rich glycoprotein (HRG), histone H1.5 (H15), histone H2B type 1-L (H2B1L), immunoglobulin gamma-1 heavy chain (IGG1), poly(rC)-binding protein 1 (PCBP1), and receptor-type tyrosine-protein phosphatase eta (PTPRJ).

7. Use of a kit for diagnosing xerostomia, the kit comprising an agent for measuring an expression level of nucleobindin-2 (NUCB2) gene, protein or fragment thereof, wherein an expression level of NUCB2 gene, protein or fragment thereof is decreased, as compared with that of a normal control group.

8. The use of claim 7, further comprising an agent for measuring an expression level of at least one gene, protein or fragment thereof selected from the group consisting of immunoglobulin kappa variable 1-8 (KV108), Golgi apparatus protein 1 (GSLG1), protein FAM3B (FAM3B), mucin-21 (MUC21), Ras-related protein Rab-11A (RB11A), lysozyme C (LYSC), calmodulin-like protein 3 (CALL3), alpha-1B-glycoprotein (A1BG), alpha-1-acid glycoprotein 1 (A1AG1), calreticulin (CALR), plasminogen (PLMN), extracellular matrix protein 1 (ECM1), annexin A6 (ANXA6), WD repeat-containing protein 1 (WDR1), leukocyte elastase inhibitor (ILEU), alpha-2-HS-glycoprotein (FETUA), myeloid cell nuclear differentiation antigen (MNDA), matrix metalloproteinase-9 (MMP9), neutrophil gelatinase-associated lipocalin (NGAL), D-dopachrome decarboxylase (DOPD), neutrophil collagenase (MMP8), programmed cell death 6-interacting protein (PDCD6IP), prostaglandin reductase 1 (PTGR1), FAM3D, Zymogen granule protein 16 homolog B (ZG16B), alpha-1-antitrypsin (A1AT), antithrombin-III (ANT3), ATP synthase subunit alpha (ATPA, mitochondrial), histidine-rich glycoprotein (HRG), histone H1.5 (H15), histone H2B type 1-L (H2B1L), immunoglobulin gamma-1 heavy chain (IGG1), poly(rC)-binding protein 1 (PCBP1), and receptor-type tyrosine-protein phosphatase eta (PTPRJ).

9. A method for diagnosing xerostomia, the method comprising measuring an expression level of nucleobindin-2 (NUCB2) gene, protein or fragment thereof in a saliva sample isolated from a subject suspected of having xerostomia;
comparing the measured expression level with an expression level of a normal control group; and
determining the subject as having xerostomia, when the expression level of NUCB2 gene, protein or fragment thereof is decreased, as compared with that of the normal control group.

10. The method of claim 9, wherein the measuring comprises incubating the saliva sample with an antibody or antigen-binding fragment thereof, or an aptamer specifically binding to the protein or fragment thereof, or with a polynucleotide identical or complementary to the polynucleotide encoding the gene.

11. The method of claim 9, wherein the measuring is performed by electrophoresis, immunoblotting, enzyme-linked immunosorbent assay (ELISA), Northern blotting, polymerase chain reaction (PCR), a protein chip, immunoprecipitation, a microarray, an electron microscopy, or a combination thereof.

12. The method of claim 9, further comprising measuring an expression level of at least one gene, protein or fragment thereof selected from the group consisting of immunoglobulin kappa variable 1-8 (KV108), Golgi apparatus protein 1 (GSLG1), protein FAM3B (FAM3B), mucin-21 (MUC21), Ras-related protein Rab-11A (RB11A), lysozyme C (LYSC), calmodulin-like protein 3 (CALL3), alpha-1B-glycoprotein (A1BG), alpha-1-acid glycoprotein 1 (A1AG1), calreticulin (CALR), plasminogen (PLMN), extracellular matrix protein 1 (ECM1), annexin A6 (ANXA6), WD repeat-containing protein 1 (WDR1), leukocyte elastase inhibitor (ILEU), alpha-2-HS-glycoprotein (FETUA), myeloid cell nuclear differentiation antigen (MNDA), matrix metalloproteinase-9 (MMP9), neutrophil gelatinase-associated lipocalin (NGAL), D-dopachrome decarboxylase (DOPD), neutrophil collagenase (MMP8), programmed cell death 6-interacting protein (PDCD6IP), prostaglandin reductase 1 (PTGR1), FAM3D, Zymogen granule protein 16 homolog B (ZG16B), alpha-1-antitrypsin (A1AT), antithrombin-III (ANT3), ATP synthase subunit alpha (ATPA, mitochondrial), histidine-rich glycoprotein (HRG), histone H1.5 (H15), histone H2B type 1-L (H2B1L), immunoglobulin gamma-1 heavy chain (IGG1), poly(rC)-binding protein 1 (PCBP1), and receptor-type tyrosine-protein phosphatase eta (PTPRJ).

## Patentansprüche

1. Verwendung einer Zusammensetzung zum Diagnostizieren von Xerostomie, wobei die Zusammensetzung ein Mittel zum Messen eines Expressionsniveaus von Nucleobindin-2(NUCB2-)Gen, -Protein oder -Fragment davon umfasst, wobei ein Expressionsniveau von NUCB2-Gen, -Protein oder -Fragment davon, wie verglichen mit demjenigen einer normalen Kontrollgruppe, verringert ist.

2. Verwendung nach Anspruch 1, wobei die Zusammensetzung zum Nachweis in einer Speichelprobe ist.

3. Verwendung nach Anspruch 1, wobei das Mittel wie folgt ist:
ein Antikörper oder ein Antigenbindungsfragment davon oder ein Aptamer, das spezifisch an das Protein oder Fragment davon bindet; oder
eine Nukleinsäure, die ein Polynukleotid umfasst, das identisch mit oder komplementär zu einem Polynukleotid ist, welches das Protein oder Fragment davon kodiert.

4. Verwendung nach Anspruch 3, wobei der Antikörper ein polyklonaler Antikörper oder ein monoklonaler Antikörper ist.

5. Verwendung nach Anspruch 3, wobei die Nukleinsäure ein Primer, eine Sonde oder ein Antisense-Oligonukleotid ist.

6. Verwendung nach Anspruch 1, ferner umfassend ein Mittel zum Messen eines Expressionsniveaus von zumindest einem Gen, Protein oder Fragment davon ausgewählt aus der Gruppe bestehend aus Immunoglobulin-kappa-Variable 1-8 (KV108), Golgi-Apparat-Protein 1 (GSLG1), Protein FAM3B (FAM3B), Mucin-21 (MUC21), Ras-verwandtem Protein Rab-11A (RB11A), Lysozym C (LYSC), Calmodulin-artigem Protein 3 (CALL3), alpha-1B-Glycoprotein (A1BG), alpha-1-Säure-Glycoprotein 1 (A1AG1), Calreticulin (CALR), Plasminogen (PLMN), extrazellulärem Matrixprotein 1 (ECM1), Annexin A6 (ANXA6), WD-Wiederholung enthaltendem Protein 1 (WDR1), Leukozyten-Elastase-Inhibitor (ILEU), alpha-2-HS-Glycoprotein (FETUA), myeloischem Zellkerndifferenzierungsantigen (MNDA), Matrixmetalloproteinase-9 (MMP9), neutrophilem Gelatinase-assoziiertem Lipocalin (NGAL), D-Dopachrom-Decarboxylase (DOPD), neutrophiler Collagenase (MMP8), programmiertem Zelltod-6-interagierendem Protein (PDCD6IP), Prostaglandin-Reductase 1 (PTGR1), FAM3D, Zymogen-Granulat-Protein-16-Homolog B (ZG16B), alpha-1-Antitrypsin (A1AT), Antithrombin-III (ANT3), ATP-Synthase-Untereinheit alpha (ATPA, mitochondrial), Histidin-reichem Glycoprotein (HRG), Histon H1.5 (H15), Histon H2B Typ 1-L (H2B1L), Immunglobulin-gamma-1-Schwerkette (IGG1), poly(rC)-Bindungsprotein 1 (PCBP1) und Rezeptor-Typ-Tyrosin-Protein-Phosphatase eta (PTPRJ).

7. Verwendung eines Kits zum Diagnostizieren von Xerostomie, wobei das Kit ein Mittel zum Messen eines Expressionsniveaus von Nucleobindin-2(NUCB2-)Gen, -Protein oder - Fragment davon umfasst, wobei ein Expressionsniveau von NUCB2-Gen, -Protein oder -Fragment davon, wie verglichen mit demjenigen einer normalen Kontrollgruppe, verringert ist.

8. Verwendung nach Anspruch 7, ferner umfassend ein Mittel zum Messen eines Expressionsniveaus von zumindest einem Gen, Protein oder Fragment davon ausgewählt aus der Gruppe bestehend aus Immunoglobulin-kappa-Variable 1-8 (KV108), Golgi-Apparat-Protein 1 (GSLG1), Protein FAM3B (FAM3B), Mucin-21 (MUC21), Ras-verwandtem Protein Rab-11A (RB11A), Lysozym C (LYSC), Calmodulin-artigem Protein 3 (CALL3), alpha-1B-Glycoprotein (A1BG), alpha-1-Säure-Glycoprotein 1 (A1AG1), Calreticulin (CALR), Plasminogen (PLMN), extrazellulärem Matrixprotein 1 (ECM1), Annexin A6 (ANXA6), WD-Wiederholung enthaltendem Protein 1 (WDR1), Leukozyten-Elastase-Inhibitor (ILEU), alpha-2-HS-Glycoprotein (FETUA), myeloischem Zellkerndifferenzierungsantigen (MNDA), Matrixmetalloproteinase-9 (MMP9), neutrophilem Gelatinase-assoziiertem Lipocalin (NGAL), D-Dopachrom-Decarboxylase (DOPD), neutrophiler Collagenase (MMP8), programmiertem Zelltod-6-interagierendem Protein (PDCD6IP), Prostaglandin-Reductase 1 (PTGR1), FAM3D, Zymogen-Granulat-Protein-16-Homolog B (ZG16B), alpha-1-Antitrypsin (A1AT), Antithrombin-III (ANT3), ATP-Synthase-Untereinheit alpha (ATPA, mitochondrial), Histidin-reichem Glycoprotein (HRG), Histon H1.5 (H15), Histon H2B Typ 1-L (H2B1L), Immunglobulin-gamma-1-Schwerkette (IGG1), poly(rC)-Bindungsprotein 1 (PCBP1) und Rezeptor-Typ-Tyrosin-Protein-Phosphatase eta (PTPRJ).

9. Verfahren zum Diagnostizieren von Xerostomie, wobei das Verfahren Messen eines Expressionsniveaus von Nucleobindin-2(NUCB2-)Gen, -Protein oder -Fragment davon in einer Speichelprobe isoliert von einem Subjekt, bei dem der Verdacht besteht, dass es Xerostomie aufweist;
Vergleichen des gemessenen Expressionsniveaus mit einem Expressionsniveau einer normalen Kontrollgruppe; und
Bestimmen, dass das Subjekt Xerostomie aufweist, wenn das Expressionsniveau des NUCB2-Gens, -Proteins oder -Fragments davon, wie verglichen mit demjenigen der normalen Kontrollgruppe, verringert ist, umfasst.

10. Verfahren nach Anspruch 9, wobei das Messen Inkubieren der Speichelprobe mit einem Antikörper oder Antigenbindungsfragment davon oder einem Aptamer, das spezifisch an das Protein oder Fragment davon bindet, oder mit einem Polynukleotid, das identisch mit oder komplementär zu dem Polynukleotid ist, welches das Gen kodiert, umfasst.

11. Verfahren nach Anspruch 9, wobei das Messen durch Elektrophorese, Immunblotten, Enzym-Linked Immunosorbent Assay (ELISA), Northern Blotting, Polymerasekettenreaktion (PCR), einen Proteinchip, Immunpräzipitation, ein Microarray, eine Elektronenmikroskopie oder einer Kombination davon durchgeführt wird.

12. Verfahren nach Anspruch 9, ferner umfassend Messen eines Expressionsniveaus von zumindest einem Gen, Protein oder Fragment davon ausgewählt aus der Gruppe bestehend aus Immunoglobulin-kappa-Variable 1-8 (KV108), Golgi-Apparat-Protein 1 (GSLG1), Protein FAM3B (FAM3B), Mucin-21 (MUC21), Ras-verwandtem Protein Rab-11A (RB11A), Lysozym C (LYSC), Calmodulin-artigem Protein 3 (CALL3), alpha-1B-Glycoprotein (A1BG), alpha-1-Säure-Glycoprotein 1 (A1AG1), Calreticulin (CALR), Plasminogen (PLMN), extrazellulärem Matrixprotein 1 (ECM1), Annexin A6 (ANXA6), WD-Wiederholung enthaltendem Protein 1 (WDR1), Leukozyten-Elastase-Inhibitor (ILEU), alpha-2-HS-Glycoprotein (FETUA), myeloischem Zellkerndifferenzierungsantigen (MNDA), Matrixmetalloproteinase-9 (MMP9), neutrophilem Gelatinase-assoziiertem Lipocalin (NGAL), D-Dopachrom-Decarboxylase (DOPD), neutrophiler Collagenase (MMP8), programmiertem Zelltod-6-interagierendem Protein (PDCD6IP), Prostaglandin-Reductase 1 (PTGR1), FAM3D, Zymogen-Granulat-Protein-16-Homolog B (ZG16B), alpha-1-Antitrypsin (A1AT), Antithrombin-III (ANT3), ATP-Synthase-Untereinheit alpha (ATPA, mitochondrial), Histidin-reichem Glycoprotein (HRG), Histon H1.5 (H15), Histon H2B Typ 1-L (H2B1L), Immunglobulin-gamma-1-Schwerkette (IGG1), poly(rC)-Bindungsprotein 1 (PCBP1) und Rezeptor-Typ-Tyrosin-Protein-Phosphatase eta (PTPRJ).

## Revendications

1. Utilisation d'une composition permettant de diagnostiquer la xérostomie, la composition comprenant un agent permettant de mesurer un niveau d'expression d'un gène, d'une protéine ou d'un fragment de celle-ci de la nucléobindine-2 (NUCB2), dans laquelle un niveau d'expression d'un gène, d'une protéine ou d'un fragment de celle-ci de NUCB2 est diminué par rapport à celui d'un groupe témoin normal.

2. Utilisation de la revendication 1, dans laquelle la composition est destinée à être détectée dans un échantillon de salive.

3. Utilisation de la revendication 1, dans laquelle l'agent est :
un anticorps ou un fragment de liaison à un antigène de celui-ci ou un aptamère qui se lie spécifiquement à la protéine ou à un fragment de celle-ci ; ou
un acide nucléique comprenant un polynucléotide identique ou complémentaire à un polynucléotide codant pour la protéine ou un fragment de celle-ci.

4. Utilisation de la revendication 3, dans laquelle l'anticorps est un anticorps polyclonal ou un anticorps monoclonal.

5. Utilisation de la revendication 3, dans laquelle l'acide nucléique est une amorce, une sonde ou un oligonucléotide antisens.

6. Utilisation de la revendication 1, comprenant en outre un agent permettant de mesurer un niveau d'expression d'au moins un gène, une protéine ou un fragment de celle-ci choisi dans le groupe constitué par une chaîne variable kappa d'immunoglobuline 1-8 (KV108), la protéine 1 de l'appareil de Golgi (GSLG1), la protéine FAM3B (FAM3B), la mucine-21 (MUC21), la protéine liée à Ras Rab-11A (RB11A), le lysozyme C (LYSC), la protéine 3 de type calmoduline (CALL3), l'alpha-1B-glycoprotéine (A1BG), l'alpha-1-glycoprotéine acide 1 (A1AG1), la calréticuline (CALR), le plasminogène (PLMN), la protéine de matrice extracellulaire 1 (ECM1), l'annexine A6 (ANXA6), la protéine 1 contenant des répétitions WD (WDR1), l'inhibiteur de l'élastase leucocytaire (ILEU), l'alpha-2-HS-glycoprotéine (FETUA), l'antigène de différenciation nucléaire des cellules myéloïdes (MNDA), la métalloprotéinase matricielle-9 (MMP9), la lipocaline associée à la gélatinase neutrophile (NGAL), la D-dopachrome décarboxylase (DOPD), la collagénase neutrophile (MMP8), la protéine d'interaction de mort cellulaire programmée 6 (PDCD6IP), la prostaglandine réductase 1 (PTGR1), FAM3D, l'homologue B de la protéine 16 de grain de zymogène (ZG16B), l'alpha-1-antitrypsine (A1AT), l'antithrombine-III (ANT3), la sous-unité alpha de l'ATP synthase (ATPA, mitochondriale), la glycoprotéine riche en histidine (HRG), l'histone H1.5 (H15), l'histone H2B type 1-L (H2B1L), la chaîne lourde de l'immunoglobuline gamma 1 (IGG1), la protéine 1 de liaison du poly(rC) (PCBP1), et la tyrosine-protéine phosphatase êta de type récepteur (PTPRJ).

7. Utilisation d'un kit permettant de diagnostiquer la xérostomie, le kit comprenant un agent permettant de mesurer un niveau d'expression d'un gène, d'une protéine ou d'un fragment de celle-ci de la nucléobindine-2 (NUCB2), ledit niveau d'expression d'un gène, d'une protéine ou d'un fragment de celle-ci de NUCB2 étant diminué par rapport à celui d'un groupe témoin normal.

8. Utilisation de la revendication 7, comprenant en outre un agent permettant de mesurer un niveau d'expression d'au moins un gène, d'une protéine ou d'un fragment de celle-ci choisi dans le groupe constitué par une chaîne variable kappa d'immunoglobuline 1-8 (KV108), la protéine 1 de l'appareil de Golgi (GSLG1), la protéine FAM3B (FAM3B), la mucine-21 (MUC21), la protéine liée à Ras Rab-11A (RB11A), le lysozyme C (LYSC), la protéine 3 de type calmoduline (CALL3), l'alpha-1B-glycoprotéine (A1BG), l'alpha-1-glycoprotéine acide 1 (A1AG1), la calréticuline (CALR), le plasminogène (PLMN), la protéine de matrice extracellulaire 1 (ECM1), l'annexine A6 (ANXA6), la protéine 1 contenant des répétitions WD (WDR1), l'inhibiteur de l'élastase leucocytaire (ILEU), l'alpha-2-HS-glycoprotéine (FETUA), l'antigène de différenciation nucléaire des cellules myéloïdes (MNDA), la métalloprotéinase matricielle-9 (MMP9), la lipocaline associée à la gélatinase neutrophile (NGAL), la D-dopachrome décarboxylase (DOPD), la collagénase neutrophile (MMP8), la protéine d'interaction de mort cellulaire programmée 6 (PDCD6IP), la prostaglandine réductase 1 (PTGR1), FAM3D, l'homologue B de la protéine 16 de grain de zymogène (ZG16B), l'alpha-1-antitrypsine (A1AT), l'antithrombine-III (ANT3), la sous-unité alpha de l'ATP synthase (ATPA, mitochondriale), la glycoprotéine riche en histidine (HRG), l'histone H1.5 (H15), l'histone H2B type 1-L (H2B1L), la chaîne lourde de l'immunoglobuline gamma 1 (IGG1), la protéine 1 de liaison du poly(rC) (PCBP1), et la tyrosine-protéine phosphatase êta de type récepteur (PTPRJ).

9. Procédé permettant de diagnostiquer la xérostomie, le procédé comprenant la mesure d'un niveau d'expression d'un gène, d'une protéine ou d'un fragment de celle-ci de la nucléobindine-2 (NUCB2) dans un échantillon de salive isolé à partir d'un sujet suspecté d'être atteint de xérostomie ;
la comparaison du niveau d'expression mesuré à un niveau d'expression d'un groupe témoin normal ; et
la détermination du sujet comme souffrant de xérostomie, lorsque le niveau d'expression d'un gène, d'une protéine ou d'un fragment de celle-ci de NUCB2 est diminué par rapport à celui du groupe témoin normal.

10. Procédé de la revendication 9, dans lequel la mesure comprend l'incubation de l'échantillon de salive avec un anticorps ou un fragment de liaison à l'antigène de celui-ci, ou un aptamère se liant spécifiquement à la protéine ou à un fragment de celle-ci, ou avec un polynucléotide identique ou complémentaire au polynucléotide codant pour le gène.

11. Procédé de la revendication 9, dans lequel la mesure est réalisée par électrophorèse, immunotransfert, dosage immuno-enzymatique (ELISA), transfert de type Northern, amplification en chaîne par polymérase (ACP), puce à protéines, immunoprécipitation, puce à ADN, microscopie électronique, ou une combinaison de ceux-ci.

12. Procédé de la revendication 9, comprenant en outre la mesure d'un niveau d'expression d'au moins un gène, d'une protéine ou d'un fragment de celle-ci choisi dans le groupe constitué par une chaîne variable kappa d'immunoglobuline 1-8 (KV108), la protéine 1 de l'appareil de Golgi (GSLG1), la protéine FAM3B (FAM3B), la mucine-21 (MUC21), la protéine liée à Ras Rab-11A (RB11A), le lysozyme C (LYSC), la protéine 3 de type calmoduline (CALL3), l'alpha-1B-glycoprotéine (A1BG), l'alpha-1-glycoprotéine acide 1 (A1AG1), la calréticuline (CALR), le plasminogène (PLMN), la protéine de matrice extracellulaire 1 (ECM1), l'annexine A6 (ANXA6), la protéine 1 contenant des répétitions WD (WDR1), l'inhibiteur de l'élastase leucocytaire (ILEU), l'alpha-2-HS-glycoprotéine (FETUA), l'antigène de différenciation nucléaire des cellules myéloïdes (MNDA), la métalloprotéinase matricielle-9 (MMP9), la lipocaline associée à la gélatinase neutrophile (NGAL), la D-dopachrome décarboxylase (DOPD), la collagénase neutrophile (MMP8), la protéine d'interaction de mort cellulaire programmée 6 (PDCD6IP), la prostaglandine réductase 1 (PTGR1), FAM3D, l'homologue B de la protéine 16 de grain de zymogène (ZG16B), l'alpha-1-antitrypsine (A1AT), l'antithrombine-III (ANT3), la sous-unité alpha de l'ATP synthase (ATPA, mitochondriale), la glycoprotéine riche en histidine (HRG), l'histone H1.5 (H15), l'histone H2B type 1-L (H2B1L), la chaîne lourde de l'immunoglobuline gamma 1 (IGG1), la protéine 1 de liaison du poly(rC) (PCBP1), et la tyrosine-protéine phosphatase êta de type récepteur (PTPRJ).
